# EUROPÄISCHE PATENTSCHRIFT

(11) **EP 3 114 218 B1**
(45) Veröffentlichungstag und Bekanntmachung des Hinweises auf die Patenterteilung: **28.06.2017**
(21) Anmeldenummer: 15707155.6
(22) Anmeldetag: 03.03.2015
(51) Int. Cl.: C12N 9/10, C12N 9/14, C12N 9/88, C12N 9/90, C08L 33/04, C12N 9/98, C12N 11/16, C08L 33/12

(54) **GRANULAT ENTHALTEND EIN ENZYM UND EIN ALKYL(METH)ACRYLAT-POLYMER**
GRANULATE CONTAINING AN ENZYME AND AN ALKYL (METH)ACRYLATE POLYMER
GRANULÉ CONTENANT UNE ENZYME ET UN POLYMÈRE D'ALKYL(MÉTH)ACRYLATE

(30) Priorität: 05.03.2014 DE 102014203964
(43) Veröffentlichungstag der Anmeldung: 11.01.2017
(73) Patentinhaber: Evonik Degussa GmbH, 45128 Essen (DE)
(72) Erfinder: HELLMERS, Frank, 48151 Münster (DE); HÜLLER, Thomas, 45772 Marl (DE); DASSINGER, Thomas, 64832 Babenhausen (DE); THUM, Oliver, 40880 Ratingen (DE)
(86) Internationale Anmeldenummer: PCT/EP2015/054361
(87) Internationale Veröffentlichungsnummer: WO 2015/132230

(56) Entgegenhaltungen:
- WO-A1-96/00773
- PISKIN K ET AL: "Radiopolymerized mixture of acrylic acid, methyl methacrylate, and polyethylene glycol as an enzyme support system", APPLIED BIOCHEMISTRY AND BIOTECHNOLOGY ; PART A: ENZYME ENGINEERING AND BIOTECHNOLOGY, HUMANA PRESS INC, NEW YORK, Bd. 10, Nr. 1 - 3, 1. Februar 1984 (1984-02-01), Seiten 73-79, XP035178029, ISSN: 1559-0291, DOI: 10.1007/BF02783737

## Beschreibung

### Gebiet der Erfindung

Gegenstand der Erfindung ist ein Granulat enthaltend
A) mindestens eine Enzym ausgewählt aus mindestens einer der Gruppen ausgewählt aus Transferasen der EC 2, Hydrolasen der EC 3, Lyasen der EC 4 und Isomerasen der EC 5,
B) mindestens ein Polymer ausgewählt aus C₁-C₁₀-Alkylacrylat-Polymer, C₁-C₁₀-Alkylmethacrylat-Polymer und C₁-C₁₀-Alkylacrylat-C₁-C₁ₒ-Alkylmethacrylat-Copolymer, bevorzugt C₁-C₁₀-Alkylacrylat-C₁-C₁₀-Alkylmethacrylat-Copolymer und
C) mindestens ein anorganisches Trägermaterial.

### Stand der Technik

Die Isomerisierung von Saccharose mit Hilfe von immobilisierten Ganzzellen, Zellextrakten oder aufgereinigten Enzymen ist mehrfach in der Literatur beschrieben (Südzucker AG, DE3038219C2; Mitsui Sugar Co., US4386158). Nachfolgend wird beispielhaft die enzymatische Umsetzung zu Isomaltulose sowie den Nebenprodukten Trehalulose, Glukose, Fruktose und geringen Anteilen an Oligosacchariden betrachtet. Die Verwendung von aktiven freien Zellen führt zu erhöhten Produktaufreinigungskosten und geringeren Ausbeuten (Landbauforschung Völkenrode (2002) SH 241:75-80). Aus diesem Grund erfolgt üblicherweise eine Immobilisierung des biokatalytisch aktiven Materials.

Nahezu alle bekannten Immobilisierungsverfahren sind für die biokatalytische Isomerisierung von Saccharose beschrieben, wie beispielsweise die adsorptive Bindung von biokatalytisch aktivem Material an Ionenaustauschern (Danisco AS, EP0915986B1), der Einschluss in unterschiedliche synthetische (Bayer AG, DE3416140A1) oder natürliche Polymere (Südzucker AG, DE3038219C2; Mitsui Sugar Co., US4386158). Eine Übersicht gebräuchlicher Immobilisierungsverfahren ist in der Literatur aufgeführt (Topics in Current Chemistry, Vol. 200:95-126).

Für den aufgeführten Prozess hat sich insbesondere der Einschluss von intakten Zellen bzw. nicht aufgereinigten Fermentationsbrühen in Alginat-Polymeren etabliert (Südzucker AG, DE3038219C2; Mitsui Sugar Co., US4386158). Die Immobilisate werden durch eine Vertropfung einer Suspension aus Zellmaterial oder aufgereinigten Enzymen sowie einer Alginat-Lösung in eine Lösung, welche Calcium-Chlorid Ionen enthält, hergestellt (Bioresource Technology (2009), Vol.100:4252-4256). Nachtteilig zeigt sich hierbei, dass die so hergestellten Immobilisate zu einem Großteil aus Wasser bestehen. Eine Lagerung bzw. Vorhaltung kann aus diesem Grund nur feucht erfolgen, da eine Trocknung zu irreversiblen Schäden in der Immobilisatstruktur führt. Eine feuchte Vorhaltung fördert eine Belastung mit Fremdkeimen, welche die festgelegten Grenzen nach Verordnung (EG) Nr. 178 / 2002 über Höchstgehalte für bestimmte Kontaminationen in Lebensmitteln nicht überschreiten darf. Mit Fremdkontaminationen ist bei einer erhöhten Wasseraktivität (a_{w}-Wert > 0,65) zu rechnen (s. Schimmelpilze: Vorkommen, Gesundheitsgefahren, Schutzmassnahmen. Wolfgang Mücke, Christa Lemmen. 2004. Ecomed Medizin, Verlagsgruppe Hüthig Jehle Rehm GmbH). Alginat Immobilisate erweisen sich zudem als instabil, insbesondere unter dem Einfluss von bereits geringen Kation-Konzentrationen, wie sie z.B. in gepufferten Lösungen vorliegen (Focus on Biotechnology, Vol.8B:375-405). Aus diesem Grund wird üblicherweise eine Stabilisierung durch zusätzliche Quervernetzung unter Verwendung von Glutaraldehyd und Polyethylenimin erreicht (Mitsui Sugar Co., US4386158; Bioresource Technology (2009), Vol.100:4252-4256). Nachteilig ist hierbei insbesondere eine Verringerung der enzymatischen Aktivität durch den Einsatz von Glutaraldehyd (Process Biochemistry (2006) Vol.41:2035-2040). Ebenfalls nachteilig sind erhöhte Entsorgungsaufwendungen (Gefährlicher Abfall nach Abfallverzeichnis-Verordnung (AVV) (Stand 10.12.2001)) durch den Einsatz der Quervernetzer.

Die Herstellung von trocknungsstabilen, biokatalytisch aktiven Granulaten ist in der Literatur mehrfach beschrieben. In Lipid Sci. Technol. - 2003. - Bd. 105. - S. 318-321 wird ein Prozess zur Immobilisierung von *Candida antarctica* Lipase B zur Ester Produktion gezeigt, welcher allerdings aufgrund der Bindemittelwahl (Maltodextrin) in wässrigen Prozessen nicht stabil ist. In Novo Nordisk AS, WO9522606 wird die Verwendung des Bindemittels Polyvinylpyrrolidone (Kollidon K25, BASF) zur Enzymgranulierung beschrieben. Allerdings ist diese Kombination in wässrigen Lösungen nicht stabil.

Glatt Ingenieurtechnik GmbH, EP1595942A1, beschreibt eine Mikroorganismen enthaltende Präparation, die nachfolgend mit einem Schutzüberzug durch Coating, beispielsweise durch Schellack, zur Stabilisierung versehen wird. Sinn dieses Coatingverfahrens ist es, die Präparate vor Medien wie z.B. Wasser oder Luftsauerstoff zu schützen. Daher sind solche Präparate per se nicht geeignet zur Verwendung in Biotransformationen, wo ein intensiver Stofftransfer zwischen Medium und Biokatalysator erzielt werden muss.

Aufgabe der Erfindung ist daher die Bereitstellung neuartiger Enzympräparate und Verfahren zur Herstellung derselben, die mindestens einen der zuvor genannten Nachteile des Standes der Technik überwinden. Insbesondere ist die Aufgabe die Herstellung von lagerstabilen, feuchtigkeitsunempfindlichen Enzympräparaten mit hoher apparenter Enzymaktivität und verbesserter mechanischer Stabilität.

### Beschreibung der Erfindung

Überraschend wurde gefunden, dass durch die Verwendung einer Mischung aus einem anorganischen Trägermaterial mit einer vorzugweise hydrophilen Oberfläche wie z.B. gefällten Kieselsäuren, einem C₁-C₁₀-Alkyl(meth)acrylat-(Co-)Polymer, sowie Enzymen unter Verwendung von bekannten Granulierungsverfahren wie Extrusion, Präparate mit hoher biokatalytischer Aktivität herstellt werden können.

Gegenstand der Erfindung ist somit ein Granulat enthaltend
A) mindestens eine Enzym ausgewählt aus mindestens einer der Gruppen ausgewählt aus Transferasen der EC 2, Hydrolasen der EC 3, Lyasen der EC 4 und Isomerasen der EC 5,
B) mindestens ein Polymer ausgewählt aus C1-C10-Alkylacrylat-Polymer, C1-C10-Alkylmethacrylat-Polymer und C1-C10-Aikyiacrylat-C1-C10-Alkylmethacrylat-Copolymer, bevorzugt C1-C10-Alkylacrylat-C1-C10-Alkylmethacrylat-Copolymer und
C) mindestens ein anorganisches Trägermaterial.

Ein weiterer Gegenstand der vorliegenden Erfindung ist ein Verfahren zur Herstellung der erfindungsgemäßen Granulate.

Ein Vorteil der vorliegenden Erfindung ist es, dass die Granulate trocknungsstabil sind. Ein weiterer Vorteil der vorliegenden Erfindung ist es, dass die Granulate lagerstabil sind. Noch ein Vorteil der vorliegenden Erfindung ist es, dass die Granulate kontaminationsarm vorgehalten werden können.

Ein weiterer Vorteil der vorliegenden Erfindung ist es, dass die Granulate mechanisch stabil sind.

Noch ein Vorteil der vorliegenden Erfindung ist es, dass die Granulate keine Gefahrstoffe zur zusätzlichen Quervernetzung wie Glutaraldehyd oder Polyethylenimin benötigen.

Ein weiterer Vorteil der vorliegenden Erfindung ist es, dass die Granulate eine hohe Stabilität auch in wässrigen Medien aufweisen.

Die vorliegende Erfindung wird nachfolgend beispielhaft beschrieben, ohne dass die Erfindung auf diese beispielhaften Ausführungsformen beschränkt sein soll.

Die im Zusammenhang mit der vorliegenden Erfindung angeführten Accession-Nummern entsprechen den ProteinBank Datenbank-Einträgen des NCBI mit Datum vom 01.10.2013; in der Regel wird vorliegend die Versionsnummer des Eintrages durch ".Ziffer" wie beispielsweise ".1", kenntlich gemacht.

Werden im Rahmen der vorliegenden Beschreibung Dokumente zitiert, so soll deren Inhalt vollständig zum Offenbarungsgehalt der vorliegenden Erfindung gehören.

Alle angegebenen Prozent (%) sind, wenn nicht anders angegeben, Massenprozent.

Das erfindungsgemäße Granulat ist bevorzugt ein festes.

Es weist vorteilhafterweise eine Halbwerts-Partikelgröße d50 [µm] im Bereich von 100 bis 2000 µm, insbesondere im Bereich von 200 bis 1500 µm und speziell im Bereich von 700 bis 1300 µm auf. Der d50 [µm] Wert markiert definitionsgemäß den Punkt, bei welchem die halbe untersuche Partikelmenge größer bzw. kleiner ist. Weiterhin geben der d10 und der d90 Wert den Bereich an, bei welchem 10 % bzw. 90 % kleiner oder gleich dieser Partikelgröße sind. Die Halbwerts-Partikelgröße d50 [µm], sowie der d10 [µm] und der d90 [µm] Wert des Partikelkollektivs werden hierbei mit Hilfe eines Camsizers® der Firma Retsch (Haan, Deutschland) über das Verfahren der dynamischen Bildanalyse unter Wahl der Standardeinstellungen ermittelt (ISO 13322-2:2006). Das angewandte Messverfahren liefert eine detaillierte Partikelgrößenanalyse des gesamten Partikelkollektivs, welche als Verteilungsdichtefunktion dargestellt wird. In der Verteilungsdichtefunktion ist der d50 Wert als Abszissenwert des größten Maximums zu entnehmen.

Bevorzugt sind Partikelkollektive einer engen Partikelgrößenverteilung zu wählen. Diese sind dadurch gekennzeichnet, dass der d10 [µm] Wert des Partikelkollektivs nicht kleiner als 50% und der d90 [µm] nicht größer als 150% der Halbwerts-Partikelgröße d50 [µm] sind.

Das erfindungsgemäße Granulat enthält ganz besonders bevorzugt mindestens ein Enzym ausgewählt aus mindestens einer der Gruppen ausgewählt aus Glycosyltransferasen der EC 2.4, Fructan-ß-fructosidasen der EC 3.2.1.80, β-Galactosidase der EC 3.2.1.23, Invertasen der EC 3.2.1.26, Aspartat-ß-decarboxylase der EC 4.1.1.11, Fumarat-Hydratasen der EC 4.2.1.2, Nitril-Hydratasen der EC 4.2.1.84, Aspartasen der EC 4.3.1.1, Xylose Isomerasen der EC 5.3.1.5 und Isomaltulose Synthasen der EC 5.4.99.11.

Das erfindungsgemäße Granulat enthält ganz besonders bevorzugt mindestens eine Isomaltulose Synthase der EC 5.4.99.11.

Erfindungsgemäß bevorzugte Granulate sind dadurch gekennzeichnet, dass die Isomaltulose Synthase ausgewählt ist aus der Gruppe der Proteine YP_002235756.1, WP_006325065.1, WP_012540141.1, WP_004157672.1, CCO82510.1, CCO82509.1, CCO78715.1, EKF64560.1, AAP57084.1, AAP57083.1, ACI12079.1 und ACF42098.1, sowie Proteine mit einer Polypeptidsequenz, bei der bis zu 60 %, bevorzugt bis zu 25 %, besonders bevorzugt bis zu
15 % insbesondere bis zu 10, 9, 8, 7, 6, 5, 4, 3, 2, 1 % der Aminosäurereste gegenüber den vorgenannten Bezugssequenzen durch Deletion, Insertion, Substitution oder eine Kombination davon verändert sind und die noch mindestens 50 %, bevorzugt 65 %, besonders bevorzugt
80 %, insbesondere mehr als 90 % der Aktivität des Proteins mit der entsprechenden, vorgenannten Bezugssequenz besitzen, wobei unter 100 % Aktivität des Bezugsprotein die pro Zeiteinheit umgesetzte Stoffmenge an Saccharose bezogen auf die eingesetzte Bezugsenzymmenge zu dem entsprechenden Isomaltulose verstanden wird. Die Aktivität kann wie in Beispiel 1 beschrieben bestimmt werden.

Die Isomaltulose Synthase der EC 5.4.99.11 kann erfindungsgemäß in jeglicher erdenklichen Form in den erfindungsgemäßen Granulaten enthalten sein, so zum Beispiel in Form von Ganzzellen, aufgeschlossenen Zellen, Zellextrakten oder aufgereinigt.

Es ist erfindungsgemäß bevorzugt, dass die Isomaltulose Synthase in Form von Ganzzellkatalysatoren enthalten ist.

Es ist erfindungsgemäß alternativ bevorzugt, dass die Isomaltulose Synthase in Form von aufgeschlossenen Zellen enthalten ist.

Geeignete Ganzzellkatalysatoren sind beispielsweise in der
EP 0625578 als *Protaminobacter rubrum* (, insbesondere CBS 574.77), *Serratia plymuthica* (, insbesondere ATCC 15928), *Serratia marescens* (, insbesondere NCIB 8285), *Leuconostoc mesenteroides* (, insbesondere NRRL-B 512 F, insbesondere ATCC 1083 a) und *Erwinia rhapontici* (, insbesondere NCPPB 1578), in der
EP 0392556 und EP1257638 als *Klebsiella terrigena* JCM 1687, *Klebsiella sp.* No. 88 (FERM BP-2838) und *Klebsiella singaporiensis* LX3 and LX21 und in der
EP1328647 als *Pantoea dispersa* UQ68J beschrieben.

Das erfindungsgemäße Granulat enthält mindestens ein Polymer ausgewählt aus C₁-C₁₀-Alkylacrylat-Polymer, C₁-C₁₀-Alkylmethacrylat-Polymer und C₁-C₁₀-Alkylacrylat-C₁-C₁₀-Alkylmethacrylat-Copolymer, wobei die vorgenannten Stoffgruppen durch die Nomenklatur "C₁-C₁₀-Alkyl(meth)acrylat-(Co-)Polymer" abgedeckt werden kann. Das enthaltene Polymer übt in dem erfindungsgemäßen Granulat bevorzugt eine Funktion als Bindemittel aus.

Erfindungsgemäß bevorzugte Granulate sind dadurch gekennzeichnet, dass das Polymer ein massenmittleres Molekulargewicht von etwa 100000 bis 1500000 g/mol, bevorzugt 500000 bis 1000000 g/mol, aufweist.

Das massenmittlere Molekulargewicht wird bestimmt mittels

Gelpermeationschromatographie (GPC). Die Charakterisierung der Proben erfolgte in Tetrahydrofuran als Eluent und gegen Polystyrol als Standard nach DIN 55672-1.

In bevorzugt enthaltenen Alkyl(meth)acrylat-Polymeren und -Copolymeren weist die Alkylgruppe 1 bis 4 Kohlenstoffatome auf.

Bevorzugt enthält das erfindungsgemäße Granulat als Polymer mindestens ein C₁-C₁₀-Alkylacrylat-C₁-C₁₀-Alkylmethacrylat-Copolymer.

Erfindungsgemäß besonders bevorzugte Granulate sind dadurch gekennzeichnet, dass das Polymer ein Ethylacrylat/Methylmethacrylat-Copolymer oder

Methylacrylat/Ethylacrylat-Copolymer ist, wobei Ethylacrylat/Methylmethacrylat-Copolymer, insbesondere Poly(ethyl acrylate-co-methyl methacrylate) 2:1, besonders bevorzugt ist.

Ethylacrylat/Methylmethacrylat-Copolymere werden beispielsweise unter den Handelsnamen Kollicoat EMM 30D von der BASF AG oder unter dem Handelsnamen Eudragit NE oder Eudragit NM von Evonik Industries vertrieben.

Das erfindungsgemäße Granulat enthält mindestens ein anorganisches Trägermaterial. Als geeignete Trägermaterialen kommen alle dem Fachmann bekannten, zur Herstellung von Granulaten geeignete anorganische Trägermaterialien in Betracht. Vorzugsweise haben diese eine hydrophile Oberfläche. Beispielhafte Vertreter solcher Trägermaterialien werden in Linqiu Cao, 2006; Carrier-bound Immobilized Enzymes: Principles, Application and Design, Chapter 1. Introduction: Immobilized Enzymes: Past, Present and Prospects; Wiley beschrieben.

Erfindungsgemäß bevorzugte Granulate sind dadurch gekennzeichnet, dass das anorganische Trägermaterial ausgewählt ist aus Kieselsäuren, insbesondere gefällten Kieselsäuren, und Aluminiumsilikaten, insbesondere Zeolithen, wobei gefällte Kieselsäuren besonders bevorzugt sind.

Eingesetzt werden können als Zeolithe beispielsweise Zeolith A, Zeolith P, Zeolith X oder Mischungen daraus. Geeignete Zeolithe umfassen beispielsweise Handelsprodukte wie Wessalith® (Evonik Industries), Zeolith MAP® (ex Crosfield) oder VEGOBOND AX® (ex SASOL).

Geeignete Kieselsäuren werden zum Beispiel durch Evonik Industries als Aerosil® oder Sipernat®, zum Beispiel Sipernat 320, vertrieben.

Kieselsäuren, die sich als Füllstoffe eignen sind im Handel erhältlich unter den Namen Aerosil® oder Sipernat® (Evonik Industries).

Erfindungsgemäß bevorzugte Granulate sind dadurch gekennzeichnet, dass B) bezogen auf das Gesamtgranulat in einer Menge von 0,1 Gew.-% bis 80 Gew.-%, bevorzugt von 1 Gew.-% bis 50 Gew.-%, besonders bevorzugt von 5 Gew.-% bis 35 Gew.-%, und C) in einer Menge von 99,5 Gew.-% bis 20 Gew.-%, bevorzugt von 99 Gew.-% bis 30 Gew.-%, besonders bevorzugt von 90 Gew.-% bis 40 Gew.-%, enthalten ist.

Erfindungsgemäß bevorzugte Granulate sind dadurch gekennzeichnet, dass diese eine spezifische Aktivität von 1 bis 1000 U/g, bevorzugt 5 bis 100 U/g,, besonders bevorzugt 10 bis 40 U/g,, aufweisen. Die spezifische Aktivität (U/g) definiert hierbei die Aktivität der Sucrose Isomerase, bezogen auf das Hauptprodukt (Isomaltulose in µmol), welches pro Minute unter definierten Bedingungen (40% Saccharose (Edukt), RT, pH 6, 100 RPM), durch 1 g Granulat gebildet wird.

Ein geeignetes Verfahren zur Messung der Aktivität wird in Beispiel 2 beschrieben.

Die spezifische Aktivität der Granulate kann beispielsweise durch Erhöhung oder Erniedrigung des Gehaltes an Isomaltulose Synthase im Granulat gesteuert werden.

Ein weiterer Gegenstand der vorliegenden Erfindung ist ein Verfahren zur Herstellung eines Granulates umfassend die Verfahrensschritte
1) Bereitstellen mindestens einer Isomaltulose Synthase der EC 5.4.99.11, mindestens eines Polymers ausgewählt aus C₁-C₁₀-Alkylacrylat-Polymer, C₁-C₁₀-Alkylmethacrylat-Polymer und C₁-C₁₀-Alkylacrylat-C₁-C₁₀-Alkylmethacrylat-Copolymer, bevorzugt C₁-C₁₀-Alkylacrylat-C₁-C₁₀-Alkylmethacrylat-Copolymer und mindestens eines anorganisches Trägermaterials,
2) Herstellung eines Teiges beinhaltend die Komponenten des Verfahrensschrittes 1) und
3) Granulieren des Teiges.

In Verfahrensschritt 1) des erfindungsgemäßen Verfahrens werden bevorzugt die Isomaltulose Synthasen, Polymere und Trägermaterialien eingesetzt, die in bevorzugten erfindungsgemäßen Granulaten bevorzugt enthalten sind.

In Verfahrensschritt 2) des erfindungsgemäßen Verfahrens werden die Komponenten homogenisiert.

Bevorzugt enthält der Teig in Verfahrensschritt 2) bezogen auf den Gesamtteig B) in einer Menge von 0,1 Gew.-% bis 80 Gew.-%" bevorzugt von 1 Gew.-% bis 50 Gew.-%, besonders bevorzugt von 5 Gew.-% bis 35 Gew.%, und C) in einer Menge von 99,5 Gew.-% bis 20 Gew.-%, bevorzugt von 99 Gew.-% bis 30 Gew.-%, besonders bevorzugt von 90 Gew.-% bis 40 Gew.-%. Neben den vorgenannten Bestandteilen kann der Teig Wasser in einer Menge, die eine ausreichende Homogenisierung der den Teig bildenden Bestandteile gewährleistet, enthalten.

In Verfahrensschritt 3) kann die Granulierung grundsätzlich in beliebiger Weise erfolgen. Beispielsweise kann man den Teig und gegebenenfalls weitere Bestandteile wie Wasser, Puffer, stabilisierende Metallsalze, durch Extrusion, Mischergranulation, Wirbelschichtgranulation, Telleragglomeration, Sprühagglomeration, Sprühgranulation oder Kompaktierung in an sich bekannter Weise zu einem Granulat verarbeiten.

In einer bevorzugten Ausführungsform umfasst die Granulierung in einem ersten Schritt die Extrusion des wasserhaltigen Teigs, der die Komponenten A), B) und C) und gegebenenfalls weitere Bestandteile wie Puffer, stabilisierende Metallsalze umfasst. Hier ist Wasser in einer Menge enthalten, die eine hinreichende Konsistenz (Plastifizierung) des Teigs für die Extrusion gewährleistet.

Die hierfür erforderliche Wassermenge kann von einem Fachmann auf dem Gebiet der Enzymformulierung in an sich bekannter Weise ermittelt werden. Der Wasseranteil im Teig liegt zu Beginn der Granulierung typischerweise im Bereich von 10 Gew.-% bis 80 Gew.-%, insbesondere im Bereich von 15 Gew.-% bis 70 Gew.-% und speziell im Bereich von 20 Gew.-% bis 60 Gew.-%, bezogen auf das Gesamtgewicht des Teigs.

Die Herstellung des Teigs in dieser bevorzugten Ausführungsform erfolgt in Verfahrensschritt 2) in an sich bekannter Weise durch Vermischen der den Teig bildenden Bestandteile in einer geeigneten Mischvorrichtung, beispielsweise in einem üblichen Mischer oder Kneter. Dazu werden der bzw. die Feststoffe, z.B. das Trägermaterial, mit einer flüssigen Phase, beispielsweise Wasser, einer wässrige Bindemittel-Lösung oder einem wässrigen Enzymkonzentrat, intensiv vermischt. In der Regel wird man den Träger als Feststoff in den Mischer einbringen und mit einem wässrigen Enzymkonzentrat sowie mit dem Polymer, vorzugsweise in Form einer separaten wässrigen Lösung oder gelöst in dem wässrigen Enzymkonzentrat, sowie gegebenenfalls mit stabilisierenden Salz, vorzugsweise in Form einer separaten wässrigen Lösung oder Suspension, insbesondere gelöst oder suspendiert in dem wässrigen Enzymkonzentrat, vermischen. Gegebenenfalls wird man weiteres Wasser zur Einstellung der gewünschten Konsistenz des Teigs zufügen.

Vorzugsweise wird man während des Durchmischens eine Temperatur von 60°C, insbesondere von 40°C nicht überschreiten. Besonders bevorzugt beträgt die Temperatur des Teigs während des Mischens 10 bis 30°C. Gegebenenfalls wird man daher die Mischvorrichtung während der Teigherstellung kühlen.

Der so erhaltene Teig wird in dieser bevorzugten Ausführungsform in Verfahrensschritt 3) anschließend einer Extrusion, vorzugsweise einer Extrusion bei niedrigem Druck unterworfen. Das Extrudieren, insbesondere das Extrudieren bei niedrigem Druck, erfolgt in der Regel in einem Apparat, bei dem die zu extrudierende Masse (Teig) durch eine Matrize gedrückt wird. Der Lochdurchmesser der Matrize bestimmt den Teilchendurchmesser und liegt in der Regel im Bereich von 0,3 bis 2 mm und insbesondere im Bereich von 0,4 bis 1,0 mm. Geeignete Extruder sind z. B. Domextruder oder Korbextruder, die unter anderem von Firmen wie Caleva, Fitzpatrick oder Bepex vertrieben werden. Bei richtiger Konsistenz der zu granulierenden Masse ergibt sich hierbei ein nur geringer Temperaturanstieg beim Passieren der Matrize (bis ca. 20°C). Vorzugsweise erfolgt die Extrusion unter Temperaturkontrolle, d.h. die Temperatur des Teigs sollte während des Extrudierens eine Temperatur von 70°C, insbesondere 60°C nicht überschreiten. Insbesondere liegt die Temperatur des Teigs während der Extrusion im Bereich von 10 bis 40°C.

Die den Extruder verlassenden extrudierten Teigstränge zerbrechen in kurze granulatartige Partikel oder können gegebenenfalls mit Hilfe geeigneter Schneidvorrichtungen gebrochen werden. Die so erhaltenen Granulatpartikel weisen typischerweise eine homogene Korngröße, d.h. eine enge Korngrößenverteilung auf. Ferner hat es sich als vorteilhaft erwiesen, das noch feuchte Granulat vor Durchführung der Trocknung zu runden, d.h. zu spheronisieren. Hierbei wird insbesondere die Bildung unerwünschter Staubanteile im Endprodukt verringert.

Zur Rundung der feuchten Granulate geeignete Vorrichtungen sind sog. Spheronizer, die im Wesentlichen eine horizontal rotierende Scheibe, auf der die Stränglinge durch die Zentrifugalkraft an die Wand gedrückt werden, aufweisen. Die Stränglinge brechen an den durch den Extrusionsprozess vorgegebenen Mikrokerben auf, so dass zylinderförmige Teilchen mit einem Verhältnis von Durchmesser zu Länge von etwa 1:1,3 bis 1:3 entstehen. Durch die mechanische Beanspruchung im Spheronizer werden die zunächst zylinderförmigen Teilchen etwas gerundet.

Auf diese Weise erhält man ein Granulat mit einem vergleichsweise hohen Wassergehalt, der in der Regel mehr als 15 Gew.-%, beispielsweise im Bereich von 15 bis 50 Gew.-%, insbesondere im Bereich von 20 bis 45 Gew.-%, bezogen auf das Gesamtgewicht des feuchten Granulats beträgt. Erfindungsgemäß wird es daher bevorzugt in einer Weise getrocknet, dass sein Wassergehalt nicht mehr als 30 Gew.-% beträgt und vorzugsweise im Bereich von 1 bis 12 Gew.-%, insbesondere im Bereich von 3 bis 10 Gew.-% und speziell im Bereich von 5 bis 9 Gew.-% liegt.

Die Konfektionierung umfasst dementsprechend in der Regel einen Trocknungsschritt. Dieser erfolgt bevorzugt in einem Wirbelschichttrockner. Hierbei wird ein bevorzugt erwärmtes Gas, in der Regel Luft oder ein Stickstoffgasstrom von unten durch die Produktschicht geleitet. Die Gasmenge wird üblicherweise so eingestellt, dass die Teilchen fluidisiert werden und wirbeln. Durch den Wärmeübergang Gas/Teilchen wird das Wasser verdunstet. Da enzymhaltige Granulate in der Regel temperaturlabil sind, wird man darauf achten, dass die Temperatur des Granulats nicht zu hoch ansteigt, d.h. in der Regel nicht über 80°C und bevorzugt nicht über 70°C. Insbesondere liegt die Temperatur des Granulats während des Trocknens im Bereich von 10 bis 40°C. Die Trocknungstemperatur kann in einfacher Weise über die Temperatur des Gasstroms gesteuert werden. Die Temperatur des Gasstroms liegt typischerweise im Bereich von 140 bis 40°C und insbesondere im Bereich von 120 bis 60°C. Die Trocknung kann kontinuierlich und diskontinuierlich erfolgen.

Nach der Trocknung kann das Granulat noch mittels eines Siebes fraktioniert werden. Grob- und Feingut können gemahlen und in den Mischer zum Anmaischen der Granuliermasse rückgeführt werden.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

Folgende Abbildungen sind Bestandteil der Beispiele:
Figur 1: Spezifische Aktivität (U/g) bezogen auf das Hauptprodukt (Isomaltulose in µmol) welches pro Minute unter definierten Bedingungen (40 % Saccharose (Edukt), RT, pH 6, 100 RPM), durch 1 g Granulat bzw. durch eine *P. rubrum* enthaltene Fermentationsbrühe mit einer dem Granulat äquivalenter Biomasse, gebildet wird.

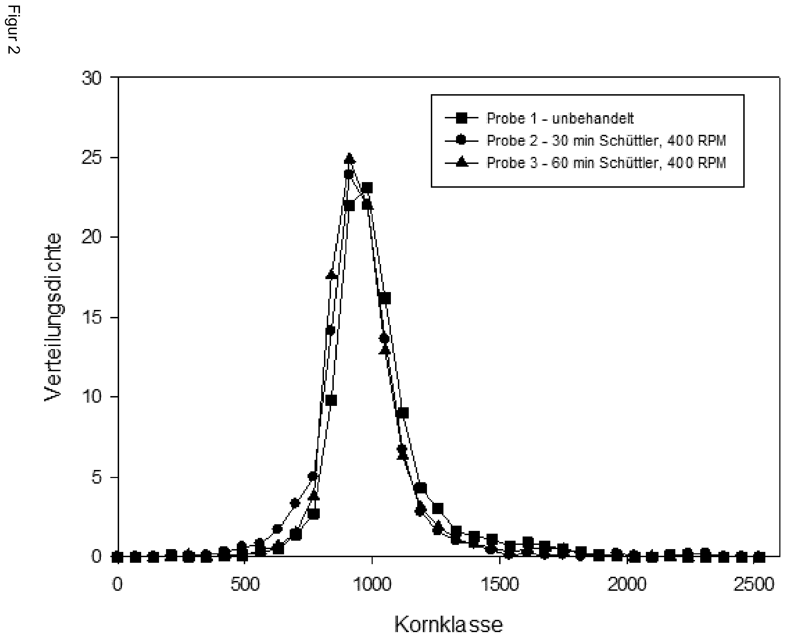

Figur 2: Darstellung der Partikelgrößenverteilung des erfindungsgemäßen Präparats (unbehandelt bzw. nach Inkubation auf einem Schüttler bei 400 RPM) als Verteilungsdichtefunktion.
Figur 3: Darstellung der Partikelgrößenverteilung des nicht erfindungsgemäßen Präparats (unbehandelt bzw. nach Inkubation auf einem Schüttler bei 400 RPM) als Verteilungsdichtefunktion.
Figur 4: Darstellung es Umsatzes von Saccharose in Fruktose und Glukose.

### Beispiele:

### Beispiel 1: Herstellung eines erfindungsgemäßen Präparats.

100 ml einer 30 Gew-%igen Dispersion eines Ethylacrylat/Methylmethacrylat-Copolymers (Eudragit NM, Evonik Industries AG) werden mit 130 ml einer an Trockenmasse 6 Gew-%igen Fermentationsbrühe von *P. rubrum* und 62 g Trägermaterial bestehend aus Sipernat 320 (Evonik Industries AG) in einem Kneter homogenisiert. Die homogene Mischung wird anschließend mit einem Extruder (Extruder 20, Caleva) extrudiert und mit einem Spheronizer (Spheronizer 250, Cavela) granuliert.

Das erhaltene Granulat wird bei Raumtemperatur über Nacht getrocknet und eine Restfeuchte von 30 % (w/w) eingestellt, ermittelt durch Trockenwaage.

Bezogen auf gesamtes Trockengewicht enthalten die Partikel 30 Gew.-% Ethylacrylat/Methylmethacrylat-Copolymer, 8 Gew.-% Fermentationstrockenmasse und 62 Gew.-% Sipernat.

### Beispiel 2: apparente Aktivität

Bestimmung der apparenten Aktivität des Präparats gemäß Beispiel 1.

Die Bestimmung der Aktivität erfolgt in drei separaten Batch-Ansätzen. Hierbei werden jeweils 1 g der getrockneten Granulate in ein 15 ml Reaktionsröhrchen gegeben und mit 10 ml 40% (w/w) Saccharose-Lösung beaufschlagt. Die Inkubation erfolgt auf einem Schüttler mit 100 RPM bei RT und einem pH-Wert von 6. Die spezifische Aktivität U/g wurde nach einer Inkubationszeit von 120 min durch die Bestimmung der Produktkonzentration erfasst. Diese wurde hierbei jeweils mit Hilfe von HPLC Analytik erfasst. Die spezifische Aktivität von *P. rubrum* enthaltener Fermentationsbrühe wurde analog, mit einer den Granulaten enthaltenen äquivalenten Biomasse, bestimmt.

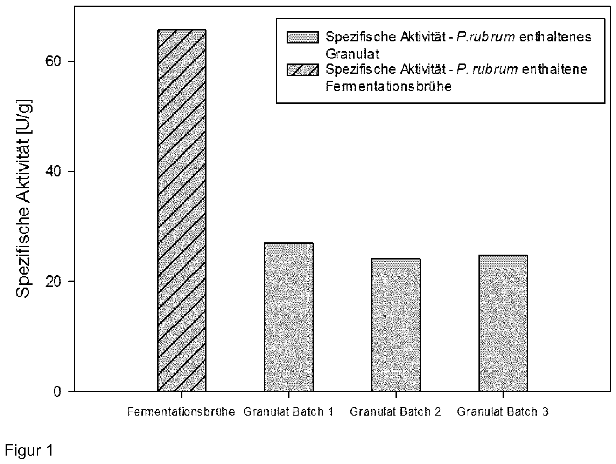

Die Ergebnisse sind in Figur 1 abgebildet.

### Beispiel 3: mechanische Stabilität

Die Beurteilung der mechanischen Stabilität des Präparats gemäß Beispiel 1 erfolgt durch die

Ermittlung des d10 [µm], d50 [µm] sowie des d90 [µm] Wertes der Partikelgrößenverteilung aus der Verteilungsdichtefunktion. Diese wurde unter Standardbedingungen mit Hilfe eines Retsch Camsizers optisch bestimmt.

Die Ermittlung der Partikelgrößenverteilung erfolgt unbehandelt sowie nach 30 bzw. 60 Minuten Inkubation in einem 50 ml Reaktionsgefäß mit jeweils 25 g Granulat und 25 g Medium auf einem Schüttler (400 rpm) im Medium und anschließender Trocknung. Insbesondere durch den nahezu unveränderten d10 [µm] Wert, welcher der Verteilungsdichtefunktion zu entnehmen ist, wird deutlich, dass das Präparat stabil ist und keine Partikelbruchstücke entstehen. Demnach haben 10 % der Partikel des Partikelkollektivs vor der Inkubation einen Durchmesser kleiner als 881 µm und nach 60 Minuten Inkubation einen Durchmesser von kleiner als 866 µm.

### Partikelgrößenverteilung - erfindungsgemäßes Präparat

| **Erfindungsgemäßes Präparat** | | **d(10) [µm]** | **d(50) [µm]** | **d(90) [µm]** |
|---|---|---|---|---|
| **Probe 1** | In Medium - 0 min | 881 | 1017 | 1267 |
| **Probe 2** | In Medium - 30 min Schüttler, 400 RPM | 818 | 980 | 1160 |
| **Probe 3** | In Medium - 60 min Schüttler, 400 RPM | 866 | 983 | 1183 |

### Beispiel 4: Herstellung eines erfindungsgemäßen Präparats enthaltend Saccharomyces cerevisiae mit dem Enzym Invertase (EC 3.2.1.26)

Die Herstellung des Präparats erfolgt gemäß Beispiel 1, wobei abweichend als biokatalytische aktive Komponente 130 ml einer an Trockenmasse 6 Gew-%igen Suspension handelsübliche Saccharomyces cerevisiae enthaltend verwendet wird. Die *Saccharomyces cerevisiae* Ganzzellen enthalten unter anderem das Enzym Invertase (EC 3.2.1.26) aus der Gruppe der Hydrolasen der EC 3, welches Saccharose in Fruktose und Glukose hydrolytisch aufspaltet. Zur Untersuchung der apparenten Aktivität wurde das Präparat in einen Festbettreaktor, welcher über seitliche Septen zur Probennahme verfügt, gegeben. Als Edukt wurde eine 40 % (w/w) Saccharose Lösung verwendet, welche auf einen pH-Wert von 6 eingestellt wurde. Die Aufgabe des Eduktes erfolgte mit einer LHSV [h-1] von 0.2, welche durch den Quotienten aus Volumenstrom [m3/h] und dem verwendeten Katalysatorvolumen [m3] gebildet wird. Um den stationären Zustand im Festbettreaktor zu erreichen, erfolgte die Probennahme nach einer Einlaufphase von 72 h. Da die Probennahme entlang der Reaktorlänge erfolgte, kann der Umsatz des Eduktes über die dimensionslose Reaktorlänge x/X [-] verfolgt werden. Die Probenzusammensetzung wurde jeweils mit Hilfe von HPLC Analytik erfasst.

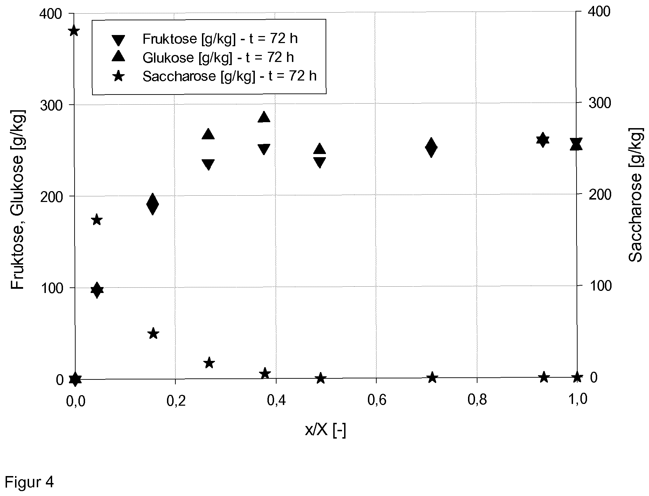

Die Ergebnisse sind in Figur 4 dargestellt.

### Vergleichsbeispiel 1 (nicht erfindungsgemäß): Herstellung eines nicht erfindungsgemäßen Präparats.

Herstellung eines Präparats gemäß WO95/22606 (Novo Nordisk A/S): *Method for production of an immobilized enzyme preparation and use of the immobilized enzyme preparation.*

65 g of Celkate T-21 werden pulverförmig in einen Hochgeschwindigkeitsmixer gegeben. Hierzu werden kontinuierlich und bei laufendem Impeller dem Pulver 25 g flüssige Fermentationsbrühe Sucrose Isomerase enthaltend zugeführt. Anschließend werden weitere 50 g flüssige Fermentationsbrühe Sucrose Isomerase enthaltend, vermengt mit 3 % (w/w) Kollidon K25 polyvinylpyrrolidone (BASF) aufgegeben. Das entstandene Granulat wird über Nacht bei Raumtemperatur getrocknet und gesiebt. Der Restfeuchtegehalt wird auf 10 % eingestellt.

### Vergleichsbeispiel 2 (nicht erfindungsgemäß): mechanische Stabilität

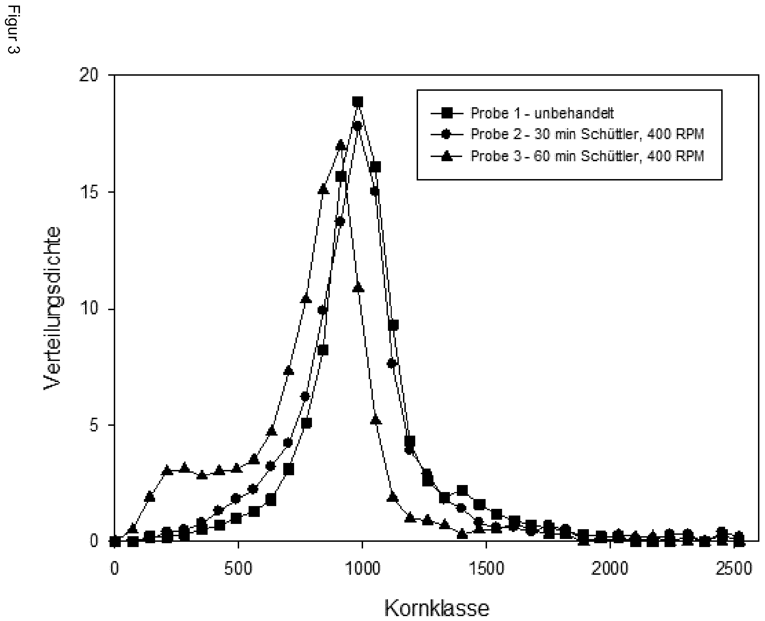

Bestimmung der mechanischen Stabilität des Präparats gemäß Vergleichsbeispiel 1. Die Messung erfolgte analog zu Beispiel 1. Ergebnisse sind in Figur 3 abgebildet. Die deutliche Verschiebung des d10 Wertes [µm] zeigt, dass vor der Inkubation 10 % des Partikelkollektivs kleiner als 784 µm und nach der Inkubation kleiner als 389 µm sind. Dies verdeutlicht, dass das nicht erfindungsgemäße Präparat durch die mechanische Beanspruchung auf dem Schüttler in kleinere Partikelbruchstücke zerfällt.

| **Nicht erfindungsgemäßes Präparat** | | **d(10) [µm]** | **d(50) [µm]** | **d(90) [µm]** |
|---|---|---|---|---|
| **Probe 1** | In Medium - 0 min | 784 | 1024 | 1355 |
| **Probe 2** | In Medium - 30 min Schüttler, 400 RPM | 695 | 1004 | 1290 |
| **Probe 3** | In Medium - 60 min Schüttler, 400 RPM | 389 | 874 | 1092 |

## Patentansprüche

1. Granulat enthaltend
A) mindestens eine Enzym ausgewählt aus mindestens einer der Gruppen ausgewählt aus Transferasen der EC 2, Hydrolasen der EC 3, Lyasen der EC 4 und Isomerasen der EC 5,
B) mindestens ein Polymer ausgewählt aus C₁-C₁₀-Alkylacrylat-Polymer, C₁-C₁₀-Alkylmethacrylat-Polymer und C₁-C₁₀-Alkylacrylat-C₁-C₁₀-Alkylmethacrylat-Copolymer, bevorzugt C₁-C₁₀-Alkylacrylat-C₁-C₁₀-Alkylmethacrylat-Copolymer und
C) mindestens ein anorganisches Trägermaterial.

2. Granulat gemäß Anspruch 1, **dadurch gekennzeichnet, dass** mindestens eine Enzym ausgewählt ist aus mindestens einer der Gruppen ausgewählt aus Glycosyltransferasen der EC 2.4, Fructan-ß-fructosidasen der EC 3.2.1.80, β-Galactosidase der EC 3.2.1.23, Invertasen der EC 3.2.1.26, Aspartat-ß-decarboxylase der EC 4.1.1.11, Fumarat-Hydratasen der EC 4.2.1.2, Nitril-Hydratasen der EC 4.2.1.84, Aspartasen der EC 4.3.1.1, Xylose Isomerasen der EC 5.3.1.5 und Isomaltulose Synthasen der EC 5.4.99.11.

3. Granulat gemäß Anspruch 1 oder 2, **dadurch gekennzeichnet**, die Isomaltulose Synthase ausgewählt ist aus der Gruppe YP_002235756.1, WP_006325065.1, W _012540141.1, W _004157672.1, CCO82510.1, CCO82509.1, CC078715.1, EKF64560.1, AAP57084.1, AAP57083.1, ACI12079.1 und ACF42098.1 sowie Proteine mit einer Polypeptidsequenz, bei der bis zu 60 % der Aminosäurereste gegenüber den vorgenannten Bezugssequenzen durch Deletion, Insertion, Substitution oder eine Kombination davon verändert sind.

4. Granulat gemäß mindestens einem der vorherigen Ansprüche, **dadurch gekennzeichnet, dass** die Isomaltulose Synthase in Form von Ganzzellkatalysatoren oder in Form von aufgeschlossenen Zellen enthalten ist.

5. Granulat gemäß mindestens einem der vorherigen Ansprüche, **dadurch gekennzeichnet, dass** das Polymer ein massenmittleres Molekulargewicht von etwa 100000 bis 1500000 g/mol, bevorzugt 500000 bis 1000000 g/mol, aufweist.

6. Granulat gemäß mindestens einem der vorherigen Ansprüche, **dadurch gekennzeichnet, dass** das Polymer Ethylacrylat/Methylmethacrylat-Copolymer, bevorzugt Poly(ethyl acrylate-co-methyl methacrylate) 2:1, ist.

7. Granulat gemäß mindestens einem der vorherigen Ansprüche, **dadurch gekennzeichnet, dass** das anorganische Trägermaterial ausgewählt ist aus Kieselsäuren, insbesondere gefällten Kieselsäuren und Aluminiumsilikaten, insbesondere Zeolithen, wobei gefällte Kieselsäuren bevorzugt sind.

8. Granulat gemäß mindestens einem der vorherigen Ansprüche, **dadurch gekennzeichnet, dass** bezogen auf das Gesamtgranulat
B) in einer Menge von 0,1 Gew.-% bis 80 Gew.-% und
C) in einer Menge von 20 Gew.-% bis 95 Gew.-%
enthalten ist.

9. Granulat gemäß mindestens einem der vorherigen Ansprüche, **dadurch gekennzeichnet, dass** es eine spezifische Aktivität von 1 bis 1000 U bezogen auf das Gewicht des Gesamtgranulates aufweist.

10. Verfahren zur Herstellung eines Granulates umfassend die Verfahrensschritte
1) Bereitstellen
mindestens einer Isomaltulose Synthase der EC 5.4.99.11,
mindestens eines Polymers ausgewählt aus C₁-C₁₀-Alkylacrylat-Polymer, C₁-C₁₀-Alkylmethacrylat-Polymer und C₁-C₁₀-Alkylacrylat-C₁-C₁₀-Alkylmethacrylat-Copolymer, bevorzugt C₁-C₁₀-Alkylacrylat-C₁-C₁₀-Alkylmethacrylat-Copolymer und
mindestens eines anorganisches Trägermaterials,
2) Herstellung eines Teiges beinhaltend die Komponenten des Verfahrensschrittes 1) und
3) Granulieren des Teiges.

11. Verfahren gemäß Anspruch 10, **dadurch gekennzeichnet, dass** in Verfahrensschritt 3) das Granulieren durch Extrusion, Mischergranulation, Wirbelschichtgranulation, Telleragglomeration, Sprühagglomeration, Sprühgranulation oder Kompaktierung erfolgt.

## Claims

1. Granules comprising
A) at least one enzyme selected from at least one of the groups selected from transferases of EC 2, hydrolases of EC 3, lyases of EC 4 and isomerases of EC 5,
B) at least one polymer selected from C₁-C₁₀-alkyl acrylate polymer, C₁-C₁₀-alkyl methacrylate polymer and C₁-C₁₀-alkyl acrylate-C₁-C₁₀-alkyl methacrylate copolymer, preferably C₁-C₁₀-alkyl acrylate-C₁-C₁₀-alkyl methacrylate copolymer and
C) at least one inorganic carrier material.

2. Granules according to Claim 1, **characterized in that** at least one enzyme is selected from at least one of the groups selected from glycosyltransferases of EC 2.4, fructan ß-fructosidases of EC 3.2.1.80, ß-Galactosidase of EC 3.2.1.23, invertases of EC 3.2.1.26, aspartate ß-decarboxylase of EC 4.1.1.11, fumarate hydratases of EC 4.2.1.2, nitrile hydratases of EC 4.2.1.84, aspartases of EC 4.3.1.1, xylose isomerases of EC 5.3.1.5 and isomaltulose synthases of EC 5.4.99.11.

3. Granules according to Claim 1 or 2, **characterized in that** the isomaltulose synthase is selected from the group YP_002235756.1, WP_006325065.1, WP_012540141.1, WP_004157672.1, CCO82510.1, CCO82509.1, CCO78715.1, EKF64560.1, AAP57084.1, AAP57083.1, ACI12079.1 and ACF42098.1, and proteins with a polypeptide sequence in which up to 60% of the amino acid radicals are modified compared to the aforementioned reference sequences by deletion, insertion, substitution or a combination thereof.

4. Granules according to at least one of the preceding claims, **characterized in that** the isomaltulose synthase is present in the form of whole-cell catalysts or in the form of disrupted cells.

5. Granules according to at least one of the preceding claims, **characterized in that** the polymer has a mass-average molecular weight of about 100 000 to 1 500 000 g/mol, preferably 500 000 to 1 000 000 g/mol.

6. Granules according to at least one of the preceding claims, **characterized in that** the polymer is ethyl acrylate/methyl methacrylate copolymer, preferably poly(ethyl acrylate-co-methyl methacrylate) 2:1.

7. Granules according to at least one of the preceding claims, **characterized in that** the inorganic carrier material is selected from silicas, in particular precipitated silicas and aluminium silicates, in particular zeolites, with precipitated silicas being preferred.

8. Granules according to at least one of the preceding claims, **characterized in that**, based on the total granules,
B) is present in an amount of from 0.1% by weight to 80% by weight and
C) is present in an amount of from 20% by weight to 95% by weight.

9. Granules according to at least one of the preceding claims, **characterized in that** they have a specific activity of 1 to 1000 U, based on the weight of the total granules.

10. Process for producing granules comprising the process steps
1) provision
of at least one isomaltulose synthase of EC 5.4.99.11,
at least one polymer selected from C₁-C₁₀-alkyl acrylate polymer, C₁-C₁₀-alkyl methacrylate polymer and C₁-C₁₀-alkyl acrylate-C₁-C₁₀-alkyl methacrylate copolymer, preferably C₁-C₁₀-alkyl acrylate-C₁-C₁₀-alkyl methacrylate copolymer and at least one inorganic carrier material,
2) production of a dough containing the components of process step 1) and
3) granulation of the dough.

11. Process according to Claim 10, **characterized in that** in process step 3) the granulation takes place by extrusion, mixer granulation, fluidized-bed granulation, pan agglomeration, spray agglomeration, spray granulation or compaction.

## Revendications

1. Granulat contenant
A) au moins une enzyme choisie dans au moins un des groupes choisis parmi les transférases d'EC 2, les hydrolases d'EC 3, les lyases d'EC 4 et les isomérases d'EC 5,
B) au moins un polymère choisi parmi le polymère d'acrylate de C₁-C₁₀-alkyle, le polymère de méthacrylate de C₁-C₁₀-alkyle et le copolymère d'acrylate de C₁-C₁₀-alkyle/méthacrylate de C₁-C₁₀-alkyle, de préférence le copolymère d'acrylate de C₁-C₁₀-alkyle/méthacrylate de C₁-C₁₀-alkyle et
C) au moins un matériau support inorganique.

2. Granulat selon la revendication 1, **caractérisé en ce qu'**au moins une enzyme est choisie dans au moins un des groupes choisis parmi les glycosyltransférases d'EC 2.4, les fructane-ß-fructosidases d'EC 3.2.1.80, la ß-Galactosidase d'EC 3.2.1.23, les invertases d'EC 3.2.1.26, l'aspartate-ß-décarboxylase d'EC 4.1.1.11, les fumarate-hydratases d'EC 4.2.1.2, les nitrile-hydratases d'EC 4.2.1.84, les aspartases d'EC 4.3.1.1, les xylose-isomérases d'EC 5.3.1.5 et les isomaltulose-synthases d'EC 5.4.99.11.

3. Granulat selon la revendication 1 ou 2, **caractérisé en ce que** l'isomaltulose-synthase est choisie dans le groupe formé par YP_002235756.1, WP_006325065.1, WP_012540141.1, WP_004157672.1, CCO82510.1, CCO82509.1, CCO78715.1, EKF64560.1, AAP57084.1, AAP57083.1, ACI12079.1 et ACF42098.1 ainsi que les protéines présentant une séquence polypeptidique dans laquelle jusqu'à 60% des radicaux d'acide aminé sont modifiés par rapport aux séquences de référence susmentionnées par délétion, insertion, substitution ou une combinaison de celles-ci.

4. Granulat selon au moins l'une quelconque des revendications précédentes, **caractérisé en ce que** l'isomaltulose-synthase est contenue sous forme de catalyseurs en cellules complètes ou sous forme de cellules digérées.

5. Granulat selon au moins l'une quelconque des revendications précédentes, **caractérisé en ce que** le polymère présente un poids moléculaire massique moyen d'environ 100.000 à 1.500.000 g/mole, de préférence de 500.000 à 1.000.000 g/mole.

6. Granulat selon au moins l'une quelconque des revendications précédentes, **caractérisé en ce que** le polymère est le copolymère d'acrylate d'éthyle/méthacrylate de méthyle, de préférence le poly(acrylate d'éthyle-co-méthacrylate de méthyle) 2:1.

7. Granulat selon au moins l'une quelconque des revendications précédentes, **caractérisé en ce que** le matériau support inorganique est choisi parmi les silices, en particulier les silices précipitées et les silicates d'aluminium, en particulier les zéolithes, les silices précipitées étant préférées.

8. Granulat selon au moins l'une quelconque des revendications précédentes, **caractérisé en ce qu'**il contient, par rapport au granulat total
B) en une quantité de 0,1 à 80% en poids et
C) en une quantité de 20 à 95% en poids.

9. Granulat selon au moins l'une quelconque des revendications précédentes, **caractérisé en ce qu'**il présente une activité spécifique de 1 à 1000 U, par rapport au poids du granulat total.

10. Procédé pour la préparation d'un granulat, comprenant les étapes de procédé :
1) mettre à disposition :
au moins une isomaltulose-synthase d'EC 5.4.99.11,
au moins un polymère choisi parmi le polymère d'acrylate de C₁-C₁₀-alkyle, le polymère de méthacrylate de C₁-C₁₀-alkyle et le copolymère d'acrylate de C₁-C₁₀-alkyle/méthacrylate de C₁-C₁₀-alkyle, de préférence le copolymère d'acrylate de C₁-C₁₀-alkyle/méthacrylate de C₁-C₁₀-alkyle et
au moins un matériau support inorganique,
2) préparer une pâte comprenant les composants de l'étape de procédé 1) et
3) granuler la pâte.

11. Procédé selon la revendication 10, **caractérisé en ce que** dans l'étape 3), la granulation a lieu par extrusion, granulation dans un mélangeur, granulation en couche tourbillonnante, agglomération dans une assiette, agglomération par pulvérisation, granulation par pulvérisation ou compactage.
